# EUROPEAN PATENT APPLICATION

(11) **EP 3 315 971 A1**
(43) Date of publication of application: **02.05.2018**
(21) Application number: 16820726.4
(22) Date of filing: 06.06.2016
(51) Int. Cl.: G01N 33/68, G01N 21/76

(54) **METHOD AND APPARATUS FOR COLLECTING SIGNAL, AND METHOD AND APPARATUS FOR TRACKING CELL BY USING LIGHT SENSITIVE CHIP**

(30) Priority: 08.07.2015 CN 201510398509
(71) Applicant: Sanghai E-Blot Photoelectric Technology Co., Ltd, Shanghai 201318 (CN)
(72) Inventor: ZHANG, Yinghao, Shanghai 200120 (CN); ZHANG, Zhihao, Shanghai 200120 (CN); ZHAN, Ketuan, Shanghai 200120 (CN); WAN, Jia, Shanghai 200120 (CN)
(74) Representative: Sun, Yiming
(86) International application number: PCT/CN2016/084913
(87) International publication number: WO 2017/005075

(57) **Abstract**

A method and apparatus for collecting signals and a method and apparatus for tracking cells by using light sensitive chip, relate to the technical field of signal collection, wherein the method for collecting signals by using light sensitive chip comprises closely fitting a luminous surface of a membrane carrying optical signals to be collected on a light sensitive chip (101), placing the light sensitive chip fitted with the membrane carrying optical signals to be collected in a dark room (102), collecting optical signals (103) by the light sensitive chip in the dark room, and processing and outputting the collected optical signals (104). The method and apparatus for collecting signals by using the light sensitive chip can collect signals by contacting the light sensitive chip to convert optical signals into digital signals so as to complete quantitative analysis.

## Description

### Technical Field

The invention relates to the technical field of information acquisition, in particular to a method and apparatus for collecting signals, and method and apparatus for tracking cells by using light sensitive chip.

### Background

Western blotting is a hybrid technique that combines high-resolution gel electrophoresis with immunochemical analysis technique. With advantages of high analysis capacity, high sensitivity and strong specificity, the western blotting is a most common method for detecting the characteristics, expression and distribution of protein, such as qualitative and quantitative detection of tissue antigen, mass measurement of peptides and antibody or antigen detection of virus.

The existing apparatus and method for collecting signals by the western blotting are as follows:
Method 1: tightly attaching a light sensitive film to an NC membrane, developing and marking them after exposing for a certain period, and displaying the image on the film. The advantages are high sensitivity and high resolution. The disadvantages are as follows:
   1. Large occupied space: specialized dark room (room), sink and sewer line are required.
   2. High cost: processing machine, cassette and consumables such as lots of light sensitive films, developing solution and fixative solution should be purchased. Washing of films may waste water.
   3. Pollution of environment: much developing solution and fixative solution are required for developing films, and films discarded due to substandard quality are produced at the same time, causing heavy metal pollution and aromatic compound pollution.
   4. Unstable image quality: researcher cannot monitor exposure extent in real time in the dark room, but can get better images only after a few attempts. Mostly, the disadvantages are either underexposure or overexposure, and waste of time and energy.
   5. Waste of time: since the current data are stored, transmitted and published in digital manner, the film images will be converted into digital images by scanning.
   6. Quantitative inaccuracy: mostly, the images which are determined as good by researchers through visual inspection have been supersaturated in gray scale in fact. So the subsequent gray scale scanning is hard to quantify accurately.
Method 2: directly photographing samples by use of light sensitive devices such as CCD. The advantage is that all disadvantages in Method 1 are overcome. The disadvantage is the loss of the advantages of film collection, that is, the sensitivity is severely reduced. The reason is as follows: for all such devices on current market, a CCD digital camera is mounted above the NC film at a certain distance for shooting images. For light energy radiated by a light source, only the ray of light within a small angle can be collected by a camera. But more than 90% energy is lost. Therefore, such devices are often photographed under strong light. Only individual brands claim that they can be used for photographing WB under low light. Compared with the film, the exposure time is greatly extended. Some brands reduce resolution by pixel binning to improve sensitivity so as to reach the sensitivity matching the film. However, mosaics appear when the image is slightly magnified, and it is hard to meet various needs.
Method 3: scanning and collecting low light signals by CCD light sensitive units which are linearly aligned. The advantage is that the collection rate of optical signals is increased to improve the sensitivity. The disadvantages are as follows: since the optical signal is collected by linear scanning and the whole image cannot be collected at the same time, time difference appears during scanning of different regions. The intensities of signals collected on different time points are not comparable because the light source is constantly attenuated over time. Many control tests are not comparable.

Therefore, the technical problem to be urgently solved by those skilled in the art at present is how to innovatively provide an effective measure to solve the existing problem and meet more demands in actual applications.

### Summary of the Invention

The technical problem to be solved in the embodiments of the invention is to provide a method and apparatus for collecting signals, and method and apparatus for tracking cells by using light sensitive chip light sensitive chip to convert the signals to be detected from optical signals into digital signals and rapidly perform quantitative analysis.

Correspondingly, the embodiments of the invention also provide a apparatus for collecting signals by using light sensitive chip and an apparatus for tracking cells to ensure implementation and application of above method.

In order to solve the problems above, the invention discloses a method for collecting signals by using light sensitive chip, comprising the following steps:
closely fitting a luminous surface of a membrane carrying optical signals to be collected on a light sensitive chip;
placing the light sensitive chip fitted with the membrane carrying optical signals to be collected in a dark room which is not affected by external light;
collecting optical signals by the light sensitive chip in the dark room;
processing and outputting the collected optical signals.

Preferably, the method for collecting optical signals by the light sensitive chip in the dark room comprises the following steps:
collecting optical signals;
observing exposure extent by a computer screen in real time;
stopping exposure when signals are accumulated to a predetermined intensity;
obtaining and saving the image produced by exposure.

Preferably, when the signal collected by the light sensitive chip is a western blotting signal, the membrane carrying optical signals to be collected is obtained by the following steps:
conducting electrophoresis for the protein to be tested;
transferring the protein from a gel after completion of electrophoresis;
transferring the protein to be tested from the gel to a polyvinylidene fluoride membrane or a nitrocellulose membrane;
sealing the POLYVINYLIDENE FLUORIDE membrane or the nitrocellulose membrane after transfer, adding primary antibody reaction resisting the protein to be tested, and adding secondary antibody HRP reaction;
treating the reacted polyvinylidene fluoride membrane or nitrocellulose membrane with chemiluminescent liquid.

Preferably, the membrane comprises nitrocellulose membrane and\or polyvinylidene fluoride membrane.

Preferably, the light sensitive chip comprises CMOS light sensitive chip and CCD light sensitive chip.

The invention also discloses an apparatus for collecting signals by using light sensitive chip, comprising:
a fitting module for closely fitting a luminous surface of a membrane carrying optical signals to be collected on a light sensitive chip;
a laying module for placing the light sensitive chip fitted with the membrane carrying optical signals to be collected in a dark room which is not affected by external light; a signal collecting module for collecting optical signals by the light sensitive chip in the dark room;
a signal processing module for processing and outputting the collected optical signals.

The invention also discloses a method for tracking cells by using light sensitive chip, comprising the following steps:
implanting cells or animals carrying luciferase on the light sensitive chip;
placing the light sensitive chip implanted with cells or animals carrying luciferase in a dark room which is not affected by external light;
collecting optical signals by the light sensitive chip in the dark room;
processing and outputting the collected optical signals.

Preferably, the method further comprises the following steps before implanting cells or animals carrying luciferase on the light sensitive chip: adding a glass layer on the light sensitive chip; and implanting the cells or animals carrying luciferase on the glass layer of the light sensitive chip.

Preferably, the method for obtaining the cells and animals carrying luciferase comprises the following steps:
constructing a reporter gene plasmid for inserting a specific fragment of target promoter into the front part of the luciferase expression sequence;
co-transfecting regulatory sequence and luciferase gene plasmid into cells or the fertilized eggs of animals;
adding luciferin to the cell culture medium.

The invention also discloses an apparatus for tracking cells by using light sensitive chip, comprising:
an implanting module for implanting cells or animals carrying luciferase on the light sensitive chip;
a laying module for placing the light sensitive chip implanted with cells or animals carrying luciferase in a dark room which is not affected by external light;
a signal collecting module for collecting optical signals by the light sensitive chip in the dark room;
a signal processing module for processing and outputting the collected optical signals.

Compared with the prior art, the embodiments of the invention comprise the following advantages:
The scheme of the invention is to closely fit the luminous surface of the membrane carrying optical signals to be collected on the light sensitive chip, place the light sensitive chip fitted with the membrane carrying optical signals to be collected in the dark room, collect optical signals by the light sensitive chip in the dark room, and process and output the collected optical signals. Collection of signals by directly contacting the light sensitive chip can collect the whole image at the same time and greatly avoid the loss of optical signals, so as to improve the sensitivity without reducing the resolution.

It keeps all advantages of three methods in the background and avoids respective disadvantages.

### Brief Description of the Drawings

In order to clearly describe the embodiments of the invention or the technical scheme in the prior art, the embodiments or drawings used in technical description will be simply introduced as follows. Apparently, the drawings described below are some embodiments of the invention. Those skilled in the art can obtain other drawings based on these drawings without creative work.
Fig. 1 is a process diagram of a method for collecting signals by using light sensitive chip;
Fig. 2 is a structural diagram of an apparatus for collecting signals by using light sensitive chip;
Fig. 3 is a process diagram of a method for tracking cells by using light sensitive chip;
Fig. 4 is a structural diagram of an apparatus for tracking cells by using light sensitive chip;

### Detailed Description of the Preferred Embodiments

In order to make objectives, technical scheme and advantages of the embodiments of the invention clearer, the technical scheme in the embodiments of the invention will be described clearly and completely in combination with the figures of the embodiments as follows. Apparently, the embodiments described are some but not all embodiments of the invention. Based on the embodiments of the invention, all other embodiments obtained without creative work by those skilled in the art shall fall within the protection scope of the invention.

### Example 1

A method for collecting signals by using light sensitive chip of the embodiments of the invention is described in detail.

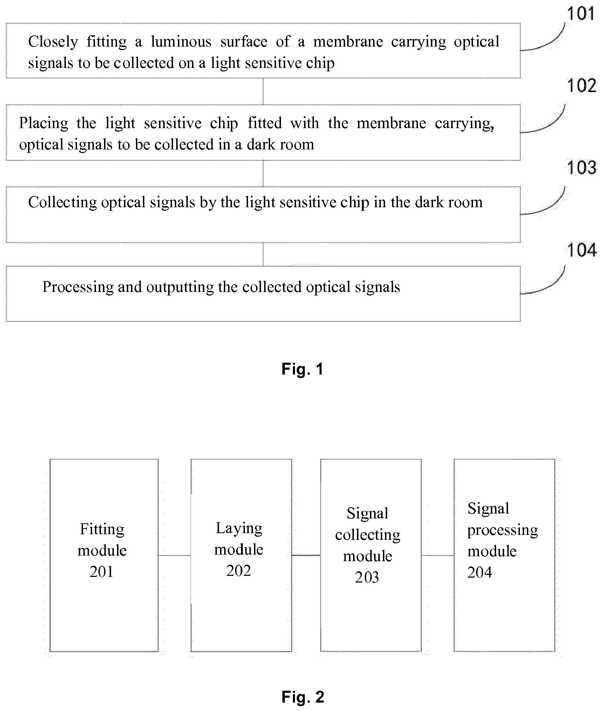

Referring to Fig. 1, a process diagram of the embodiments of a method for collecting signals by using light sensitive chip in the invention is shown, specifically comprising following steps:
Step 101, closely fitting a luminous surface of a membrane carrying optical signals to be collected on a light sensitive chip;
   In actual application, when a western blotting signal is collected by the light sensitive chip, the membrane carrying optical signals to be collected is obtained by the following steps:
   conducting electrophoresis for the protein to be tested;
   transferring the protein from a gel after completion of electrophoresis;
   transferring the protein to be tested from the gel to a polyvinylidene fluoride membrane or a nitrocellulose membrane;
   sealing the polyvinylidene fluoride membrane or the nitrocellulose membrane after transfer, adding primary antibody reaction resisting the protein to be tested, and adding secondary antibody HRP reaction;
   treating the reacted polyvinylidene fluoride membrane or nitrocellulose membrane with chemiluminescent liquid.

   In the application, the used membrane mainly comprises nitrocellulose membrane (NC membrane) and\or polyvinylidene fluoride membrane (PVDF membrane). The used light sensitive chip comprises CMOS light sensitive chip and CCD light sensitive chip. Considering that the light sensitive chip with large size has high manufacturing cost, the array of CMOS light sensitive chip and CCD light sensitive chip may be adopted to greatly reduce the cost if the chip splicing technique can achieve better effect in practice.
Step 102, placing the light sensitive chip fitted with the membrane carrying optical signals to be collected in a dark room which is not affected by external light;
   In order to avoid the influence of external light, the light sensitive chip fitted with the membrane carrying optical signals to be collected is placed in the dark room. The implementation method can be easily selected based on specific environment. The chip can be covered by a light blocking lid.
Step 103, collecting optical signals by the light sensitive chip in the dark room;
   In actual application, the method for collecting optical signals by the light sensitive chip in the dark room comprises the following steps:
   collecting optical signals;
   observing exposure extent by a computer screen in real time;
   stopping exposure when signals are accumulated to a predetermined intensity;
   obtaining and saving the image produced by exposure.
Step 104, processing and outputting the collected optical signals.

Processors such as single chip, FPGA and CPU can be used for processing and outputting the collected optical signals. Due to the mature signal processing method, the signal processing in the scheme can be easily completed, which will not be repeated herein.

The western blotting signal (WB signal) collected by the light sensitive chip is described in detail in combination with actual application as follows:
Operating principles of the western blotting:
1. Vertically separating cells or tissue extracts from the polypropylene gel using electric field;
2. Horizontally transferring the protein band to the NC membrane using electric field, this band is called Blot if its relative position does not change;
3. Sealing. Soaking the blotted membrane in BSA solution. Filling BSA solution in the areas not occupied by the protein band. Avoiding the subsequent antibodies being adsorbed in these areas, and combining the antibody with its antigen only.
4. Incubation of antibody. Hinging the antibody with HRP in advance. Soaking the antibody with the NC membrane for incubation. Specifically combining the antibody and its antigen.
5. Collection of signals. Soaking the NC membrane in the solution containing HRP substrate. Releasing fluoresce when its substrate is catalyzed with HRP. Collecting fluorescence signals by use of light sensitive film or electro-photographic system.

Steps before collection of WB optical signals are as follows:
(1) Obtaining protein sample: after bacterial-induced expression, directly splitting cells by loading buffer via electrophoresis, adding homogenized buffer to eukaryocyte, and homogenizing them for 0.5-1 min by machine or in ultrasonic room. Then centrifuging 13,000g mixture for 15min at 4°C. Taking supernatant as a sample.
(2) Electrophoresis: preparing electrophoresis gel for SDS-PAGE.
(3) Transferring: ① After electrophoresis, cutting the adhesive strips into appropriate size, balancing the strips with transfer buffer for 5min three times. ② Membrane treatment: pre-cutting the filter paper and NC membrane the same as the adhesive strips in size, and soaking them in the membrane buffer for 10min. ③ Membrane transfer: placing the membrane transfer devices including anode carbon plate, 24-layered filter paper, NC membrane, gel, 24-layered filter paper and cathode carbon plate from the bottom up, accurately aligning the filter paper, gel and NC membrane, removing bubbles in every step, and pressing by a 500g weight to absorb excess liquid on the carbon plate. Switching on with constant current 1mA/cm2 and transferring for 1.5hr. After transfer, disconnecting the power supply to take out the membrane, cutting the membrane band strip to be tested for western blotting. Dying the standard protein band, putting it in membrane staining liquid for 50s, decoloring it in 50% methanol for several times until the background is clear, washing it with double distilled water, air-drying and clamping it between two-layered filter paper for storage, and leaving it for comparison with coloration result.
(4) Immunoreaction: washing the membrane three times with 0.01 M PBST for 5min each.

Adding confining liquid, stably shaking it at the room temperature for 1hr.

Removing the coating buffer, washing the membrane with 0.01 M PBST for 5min three times.

Adding primary antibody (diluting it with 0.01M PBS according to proper dilution ratio and covering the whole membrane with liquid) and placing it for more than 12hr at 4°C. In negative control, replacing primary antibody with 1%BSA and carrying out the other steps which are the same as those in the experimental group.

Removing primary antibody and 1%BSA, and washing the membrane four times with 0.01 M PBS for 5min each.

Adding secondary antibody coupled with HRP (diluting it with 0.01 M PBS according to proper dilution ratio), stably shaking it at the room temperature for 2hr.

Removing secondary antibody, washing the membrane four times with 0.01 M PBST for 5min each.

Treating the membrane with chemiluminescent liquid, and catalyzing the substance to release fluoresce when HRP encounters the chemical substrate in liquid.

Collecting fluorescence signals by directly fitting the membrane using the digital light sensitive chip;
Taking out the membrane soaked in the chemiluminescent liquid, draining off excess liquid on the absorbent paper and fitting the luminous surface of a membrane to the digital light sensitive chip;
Pressing the membrane by flat object, and directly and closely fitting with the digital light sensitive chip;
Covering the lid and placing the digital light sensitive chip and the membrane in dark environment to avoid being polluted by external light;
Controlling the light sensitive chip by a computer and collecting chemiluminescent signals. Observing exposure extent by the computer screen in real time and stopping exposure when signals are accumulated to a proper intensity;
Obtaining and saving the image produced in exposure for quantitative and qualitative analysis.

In the embodiment, quantitative analysis can be completed by using a contact-type light sensitive chip to collect signals and converting optical signals into digital signals.

### Example 2

An apparatus for collecting signals by using light sensitive chip of the embodiments of the invention is described in detail.

Referring to Fig. 2, a structural diagram of an apparatus for collecting signals by a light sensitive chip is shown, specifically comprising:
a fitting module 201 for closely fitting a luminous surface of a membrane carrying optical signals to be collected on a light sensitive chip;
a laying module 202 for placing the light sensitive chip fitted with the membrane carrying optical signals to be collected in a dark room which is not affected by external light;
a signal collecting module 203 for collecting optical signals by the light sensitive chip in the dark room;
a signal processing module 204 for processing and outputting the collected optical signals.

Since the embodiments of the apparatus are similar to the embodiments of the method, they are just simply described. See description of the embodiments of the method for related contents.

### Example 3

A method for tracking cells by using light sensitive chip of the embodiments of the invention is described in detail.

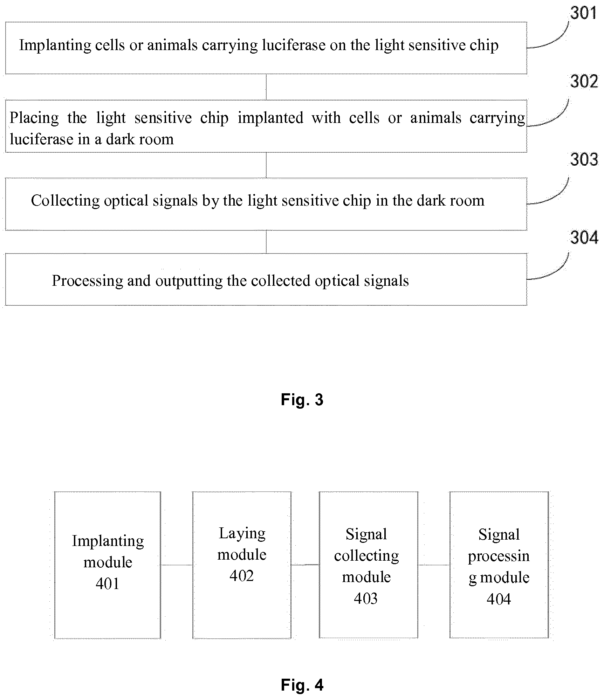

Referring to Fig. 3, a method for tracking cells by using light sensitive chip of the invention is shown, specifically comprising the following steps:
Step 301, implanting cells or animals carrying luciferase on the light sensitive chip;
Generally, a transparent protective layer or glass layer or resin layer or layer made of other materials will be covered on the light sensitive chip, and the thickness of the protective layer is less than 0.5mm.

Considering the tracking effect and the secondary use of the light sensitive chip in practice, a glass layer can be added on the light sensitive chip before implanting cells or animals carrying luciferase on the light sensitive chip, so that implant cells or animals carrying luciferase on the glass layer of the light sensitive chip and make it more convenient for later cleaning .

For the instrument using the light sensitive chip but having no illuminating module, measures should be generally taken to make cells or small animals (e.g., nematodes and drosophila) have self-luminous ability. Specifically, the common method is to transfer luciferase gene to make cells or animals and plants have self-luminous ability.

Luciferase is a protein produced from the tail of the firefly that can catalyze the reaction of luciferin with oxygen in the presence of ATP to emit fluoresce. The gene of luciferase and DNA sequence for controlling transcription are transferred to cells or animals and plants, and integrated on the host's chromosome by use of bioengineering. Some host-expressed protein molecules that have special structure and function for controlling gene expression are used to specifically bind DNA sequence for controlling transcription so as to enhance the expression of the luciferase gene.

The method for obtaining the cells and animals carrying luciferase comprises the following steps:
constructing a reporter gene plasmid for inserting a specific fragment of target promoter into the front part of the luciferase expression sequence; specifically, e.g., pGL3-basic.
co-transfecting regulatory sequence and luciferase gene plasmid into cells or the fertilized eggs of animals (transgenic animal);
adding luciferin to the cell culture medium, providing energy to catalyze the reaction of luciferin with oxygen by luciferase using ATP in the cell so as to produce fluoresce. Therefore, the instrument can track the migration path of the cells or animals. Such method can be used for animal behavior experiment.
Step 302, placing the light sensitive chip implanted with cells or animals carrying luciferase in a dark room which is not affected by external light;
Step 303, collecting optical signals by the light sensitive chip in the dark room;
Step 304, processing and outputting the collected optical signals.

### Example 4

An apparatus for tracking cells by using light sensitive chip of the embodiments of the invention is described in detail.

Referring to Fig. 4, a structural diagram of a device for tracking cells by a light sensitive chip is shown, specifically comprising:
an implanting module 401 for implanting cells or animals carrying luciferase on the light sensitive chip;
a laying module 402 for placing the light sensitive chip implanted with cells or animals carrying luciferase in a dark room which is not affected by external light;
a signal collecting module 403 for collecting optical signals by the light sensitive chip in the dark room;
a signal processing module 404 for processing and outputting the collected optical signals.

The scheme of the invention can be widely applied in collecting western blotting signals, monitoring and comparing the low light intensity in droplet array, and implanting cells on the light sensitive chip for observation of cell migration and division or dynamic process expressed by some molecules.

It should be noted that, in order to simply describe the embodiments of the method, a series of actions are combined in description. Those skilled in the art should know that the embodiments of the invention are not restricted by the sequence of the described actions, because some steps can be performed in other sequences or at the same time according to the embodiments of the invention. Those skilled in the art should also know that the embodiments described in the specification are the preferred embodiments of the invention, and the involved actions are not necessarily required in the embodiments of the invention.

Since the embodiments of the apparatus are similar to the embodiments of the method, they are just simply described. See description of the embodiments of the method for related contents.

All embodiments in the specification are described in a progressive manner, and each embodiment focuses on the difference from other embodiments, and the same or similar parts among the embodiments may be referred to each other.

The method and apparatus for collecting signals by using light sensitive chip and the method and device for tracking cells of the invention are described in detail. In the article, the specific examples are used for stating the principles and embodiments of the invention, and the above embodiments are only described for helping understand the method and the core thought of the invention. Simultaneously, for those skilled in the art, the preferred embodiments and application range may have changes based on the thought of the invention. In conclusion, the contents of the specification should not be construed as limitation to the invention.

## Claims

1. A method for collecting signals by using light sensitive chip, wherein comprising the following steps:
closely fitting a luminous surface of a membrane carrying optical signals to be collected on a light sensitive chip;
placing the light sensitive chip fitted with the membrane carrying optical signals to be collected in a dark room which is not affected by external light;
collecting optical signals by the light sensitive chip in the dark room;
processing and outputting the collected optical signals.

2. The method according to claim 1, wherein the method for collecting optical signals by the light sensitive chip in the dark room comprises the following steps:
collecting optical signals;
observing exposure extent by a computer screen in real time;
stopping exposure when signals are accumulated to a predetermined intensity;
obtaining and saving the image produced by exposure.

3. The method according to claim 1, wherein the membrane carrying optical signals to be collected is obtained by the following steps when the signal collected by the light sensitive chip is a western blotting signal:
conducting electrophoresis for a protein to be tested;
transferring the protein from a gel after completion of electrophoresis;
transferring the protein to be tested from the gel to a polyvinylidene fluoride membrane or a nitrocellulose membrane;
sealing the polyvinylidene fluoride membrane or the nitrocellulose membrane after transfer, adding primary antibody reaction resisting the protein to be tested, and adding secondary antibody HRP reaction;
treating the reacted polyvinylidene fluoride membrane or nitrocellulose membrane with chemiluminescent liquid.

4. The method according to claim 1, wherein the membrane comprises nitrocellulose membrane and\or polyvinylidene fluoride membrane.

5. The method according to claim 1, wherein the light sensitive chip comprises CMOS light sensitive chip and CCD light sensitive chip.

6. An apparatus for collecting signals by using light sensitive chip, wherein comprising:
a fitting module for closely fitting a luminous surface of a membrane carrying optical signals to be collected on a light sensitive chip;
a laying module for placing the light sensitive chip fitted with the membrane carrying optical signals to be collected in a dark room which is not affected by external light;
a signal collecting module for collecting optical signals by the light sensitive chip in the dark room;
a signal processing module for processing and outputting the collected optical signals.

7. A method for tracking cells by using light sensitive chip, wherein comprising the following steps:
implanting cells or animals carrying luciferase on the light sensitive chip;
placing the light sensitive chip implanted with cells or animals carrying luciferase in a dark room which is not affected by external light;
collecting optical signals by the light sensitive chip in the dark room;
processing and outputting the collected optical signals.

8. The method according to claim 7, wherein the method further comprises the following steps before implanting cells or animals carrying luciferase on the light sensitive chip:
adding a glass layer on the light sensitive chip; and implanting the cells or animals carrying luciferase on the glass layer of the light sensitive chip.

9. The method according to claim 7 or 8, wherein the method for obtaining the cells and animals carrying luciferase comprises the following steps:
constructing a reporter gene plasmid for inserting a specific fragment of target promoter into the front part of the luciferase expression sequence;
co-transfecting regulatory sequence and luciferase gene plasmid into cells or the fertilized eggs of animals;
adding luciferin to the cell culture medium.

10. An apparatus for tracking cells by using light sensitive chip, wherein comprising:
an implanting module for implanting cells or animals carrying luciferase on the light sensitive chip;
a laying module for placing the light sensitive chip implanted with cells or animals carrying luciferase in a dark room which is not affected by external light;
a signal collecting module for collecting optical signals by the light sensitive chip in the dark room;
a signal processing module for processing and outputting the collected optical signals.
